# EUROPEAN PATENT SPECIFICATION

(11) **EP 4 153 249 B1**
(45) Date of publication and mention of the grant of the patent: **14.02.2024**
(21) Application number: 21733178.4
(22) Date of filing: 18.05.2021
(51) Int. Cl.: A61L 2/10, A61L 2/24

(54) **ENVIRONMENTAL SANITATION ROBOT**
UMWELTSANIERUNGSROBOTER
ROBOT D'ASSAINISSEMENT DE L'ENVIRONNEMENT

(30) Priority: 18.05.2020 EP 20175271; 16.11.2020 IT 202000027402
(43) Date of publication of application: 29.03.2023
(73) Proprietor: Bazzica Engineering S.R.L., 06039 Trevi (IT)
(72) Inventor: BAZZICA, Carlo, 06039 Trevi (PG) (IT)
(74) Representative: Studio Torta S.p.A.
(86) International application number: PCT/IB2021/054266
(87) International publication number: WO 2021/234565

(56) References cited:
- KR-B1- 101 724 481
- US-A1- 2017 340 760
- US-B2- 10 406 253

## Description

### Technical field of the invention

The present invention relates to an environmental sanitation robot.

### Background of the invention

The need to sanitise environments completely and safely from viruses and bacteria is of extreme topicality and importance following the well-known events associated with the COVID 19 pandemic that is devastating the planet. In any case, even without taking into account the recent pandemic, it is estimated that viral and bacterial infections associated with general care in Europe produce about 37,000 deaths, 16 million additional hospital days and induced costs of about € 16 million. Another consequence of such infections is the extensive use of antibiotics.

Ultraviolet radiation is known to sanitise environments and eliminate pathogens (viruses and bacteria). Ultraviolet radiation of sufficient intensity destroys the DNA/RNA molecular bonds in the structure of pathogens by deactivating their synthesis.

There are state-of-the-art devices for environmental sanitation using ultraviolet radiation comprising a trolley with wheels so that it can be manually positioned in an environment and provided with a number of rectilinear ultraviolet tubes extending in a direction perpendicular to the horizontal from an upper wall of the trolley.

These devices supply the surrounding environment with a dose of energy (expressed in millijoules/cm²) that is not constant in space. For this reason, some areas of the environment may receive an excessive dose of ultraviolet energy that can damage objects in the environment (e.g. plastic objects as the ultraviolet radiation damages polymer bonds) and other areas of the environment may receive an insufficient dose of energy that does not allow pathogens to be inactivated.

In order to improve the distribution of energy so that the quantity of energy delivered to the environment is more uniform, robots have also been proposed that implement the autonomous movement of the trolley in the environment through the use of automated/automatic guided vehicles (AGVs), which are vehicles mainly used in the industrial field for moving products within a plant.

US 2017/340760 A1 discloses a system for disinfecting larger scale spaces and equipment by means of light sources, such as ultraviolet (UV) light, and more specifically UV-C light. The system is particularly useful in medical facilities for treating medical equipment or spaces, where the prevalence of pathogens requires frequent disinfecting.

KR 101 724 481 B1 discloses a UV sterilizing system comprising a UV sterilizing robot including a UV sterilizing device for emitting unidirectional UV light and capable of performing 3-degree-of-freedom movement, and a body for supporting the UV sterilizing device and being capable of autonomously moving and reading an unsterilized blind area, and configured to sterilize an object to be disinfected, and store several sorts of data related to a disinfection area; and a central server for receiving and storing several sorts of data transmitted from the UV sterilizing robot and related to the disinfection area for performing a disinfection operation, and integrally and remotely controlling a plurality of UV sterilizing robots. The UV sterilizing device comprises a lamp unit for generating UV light; a reflecting unit formed in a semi-cylindrical shape to wrap die lamp unit in a length direction, and inducing a directional conversion to enable UV light emitted from the lamp unit to be emitted in one single direction; a support unit vertically disposed in a length direction on the body, and capable of longitudinally moving and rotating in a first rotation direction; and a connecting unit for connecting the lamp unit and the support unit, and capable of rotating in a second rotation direction.

### Object and summary of the invention

The Applicant has experienced that the robots with autonomous trolleys do not always reach all areas of the surrounding environment.

Aim of the present invention is to provide an environmental sanitation robot which enables a substantially uniform amount of energy to be radiated in the environment so that the ultraviolet radiation can exert the desired anti-bacterial and anti-viral effect in all areas of the environment, even those which are difficult to reach.

This aim is achieved by the present invention, which relates to an environmental sanitation robot, as claimed in the appended claims.

### Brief Description of the drawings

Figure 1 shows a perspective view of an environmental sanitation robot according to the present invention;
Figures 2 and 3 show side and front views of the environmental sanitation robot of Figure 1;
Figure 4 shows an enlarged perspective view of a detail of the environmental sanitation robot of Figure 1 in a first operating position;
Figure 5 shows the detail of Figure 4 in a second operating position;
Figure 6 shows a perspective view of a further embodiment of the environmental sanitation robot according to the present invention;
Figures 7 and 8 show side and front views of the environmental sanitation robot of Figure 6.

### Detailed description of preferred embodiments of the invention

The present invention will now be described in detail with reference to the accompanying drawings to enable a skilled person to realize and use it. Various modifications to the embodiments presented shall be immediately clear to persons skilled in the art and the general principles disclosed herein could be applied to other embodiments and applications but without thereby departing from the scope of protection of the present invention as defined in the appended claims. Therefore, the present invention should not be considered limited to the embodiments described and shown, but should be granted the widest protective scope in accordance with the features described and claimed.

Where not otherwise defined, all the technical and scientific terms used herein have the same meaning commonly used by persons of ordinary skill in the field pertaining to the present invention. In the event of a conflict, this description, including the definitions provided, shall be binding. Furthermore, the examples are provided for illustrative purposes only and as such should not be considered limiting.

In order to facilitate understanding of the embodiments described herein, reference will be made to some specific embodiments and a specific language will be used to describe them. The terminology used herein is for the purpose of describing only particular embodiments, and is not intended to limit the scope of the present invention.

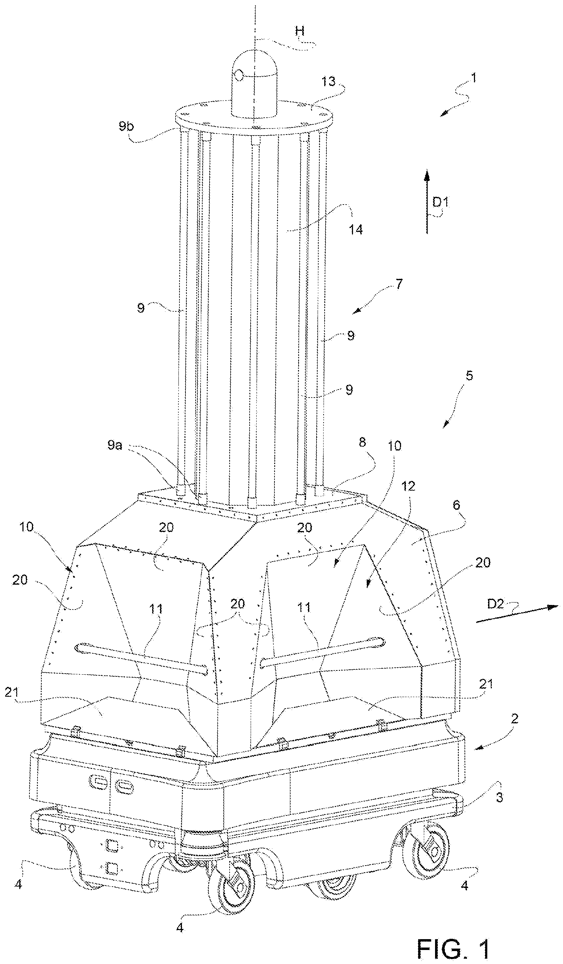

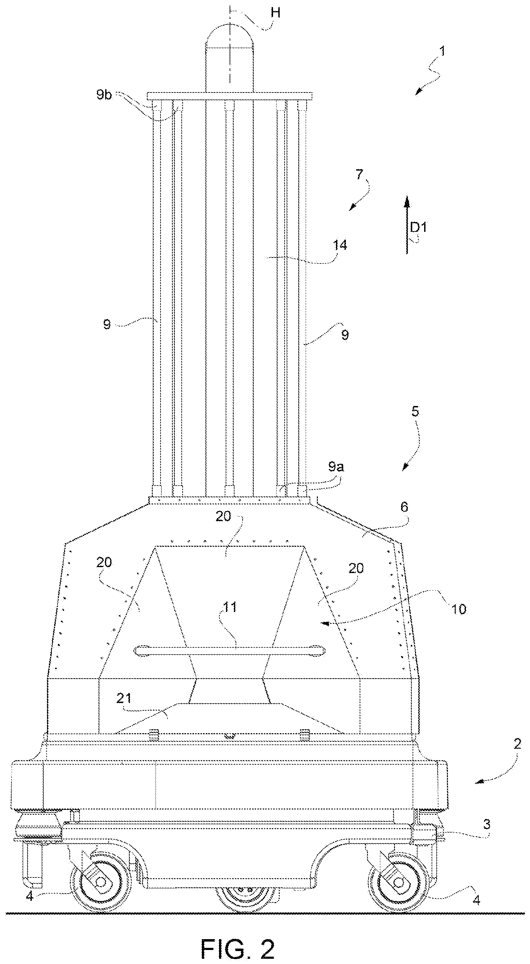

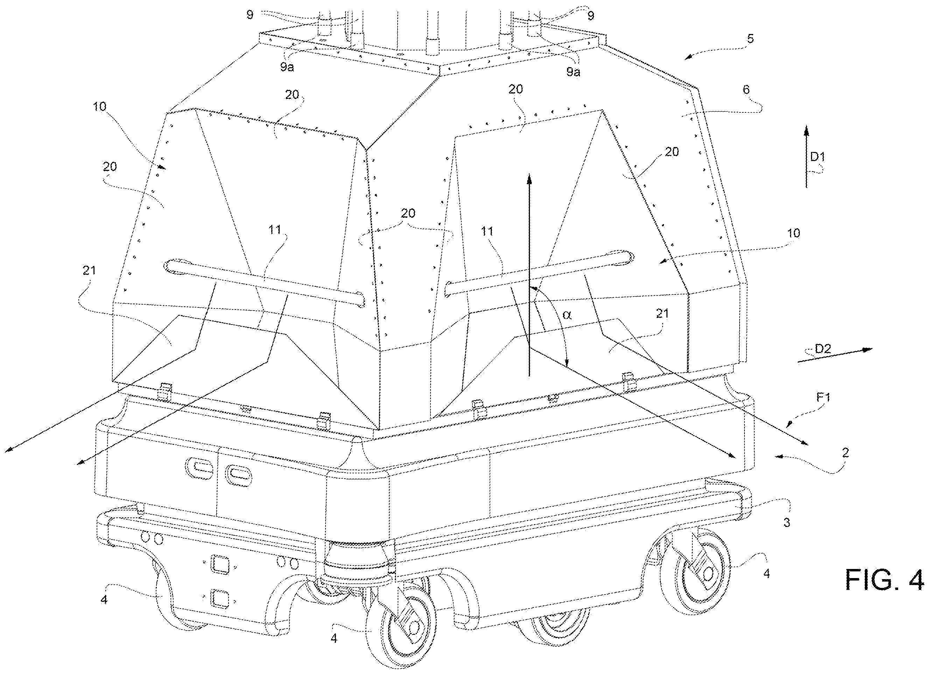

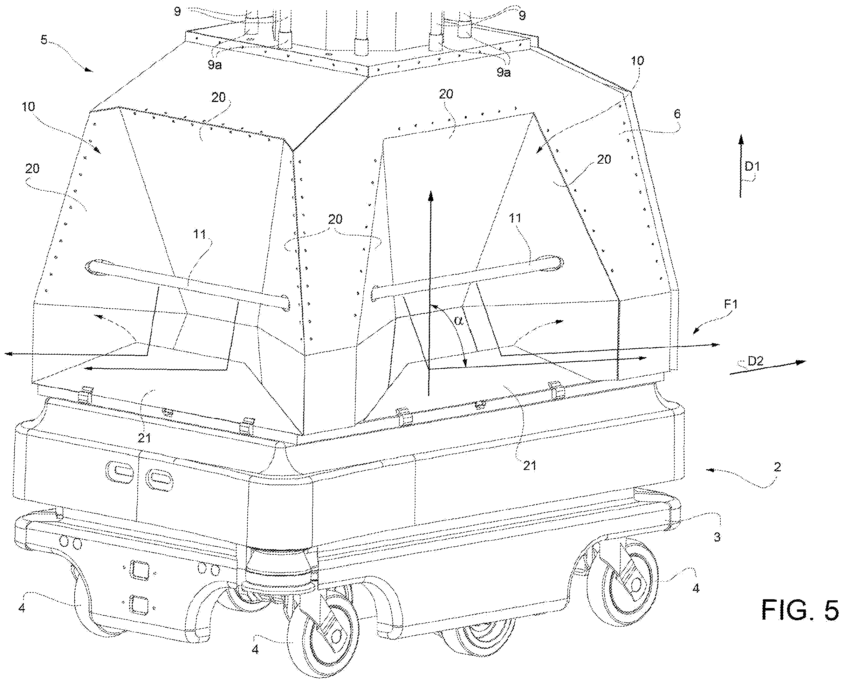

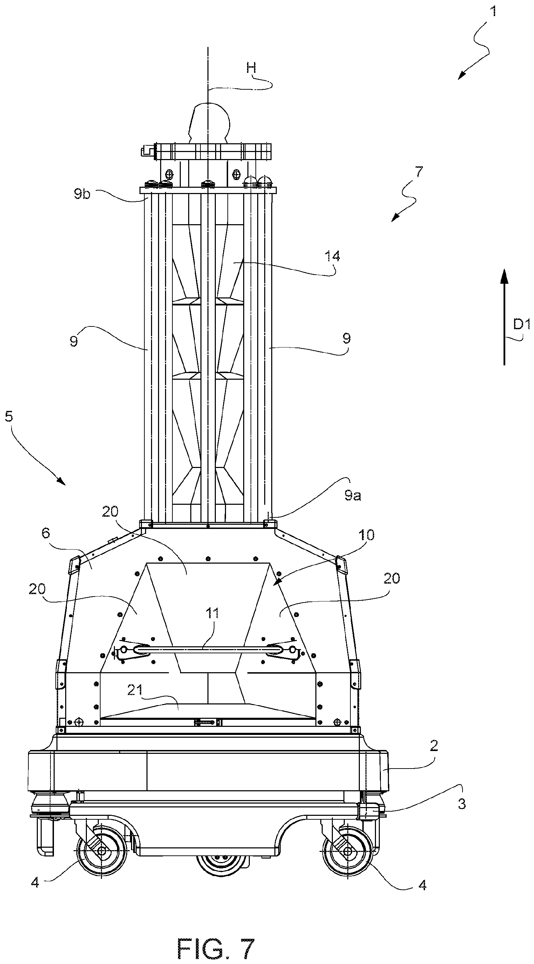

In Figures 1 to 8 reference numeral **1** denotes an automated guided robot for environmental sanitisation. The robot **1** has an axis **H,** extending in a vertical direction **D1**, and comprises an automated/automatic guided vehicle (AGV) **2,** which, as exemplarily shown in Figures 1 to 8, has an approximately parallelepiped shape and comprises a trolley **3** with four wheels **4,** at least one pair of which is motorised, for moving the automated guided vehicle **2** in the environment to be sanitised.

The robot **1** further comprises an ultraviolet radiation device **5,** which is mounted on the automated guided vehicle **2** and comprises a box frame **6** and at least one ultraviolet radiation generator **10** carried by the frame **6.**

Preferably, the frame **6** has a polyhedral shape and is delimited by a plurality of side faces, one or more of which are each conveniently provided with a respective ultraviolet radiation generator **10.** In the embodiment shown, the frame **6** has four side faces, each of at least one pair of which, conveniently both, is provided with a respective ultraviolet radiation generator **10.**

Conveniently, all of the ultraviolet radiation generators **10** are identical and hence only one of them will be described hereinafter.

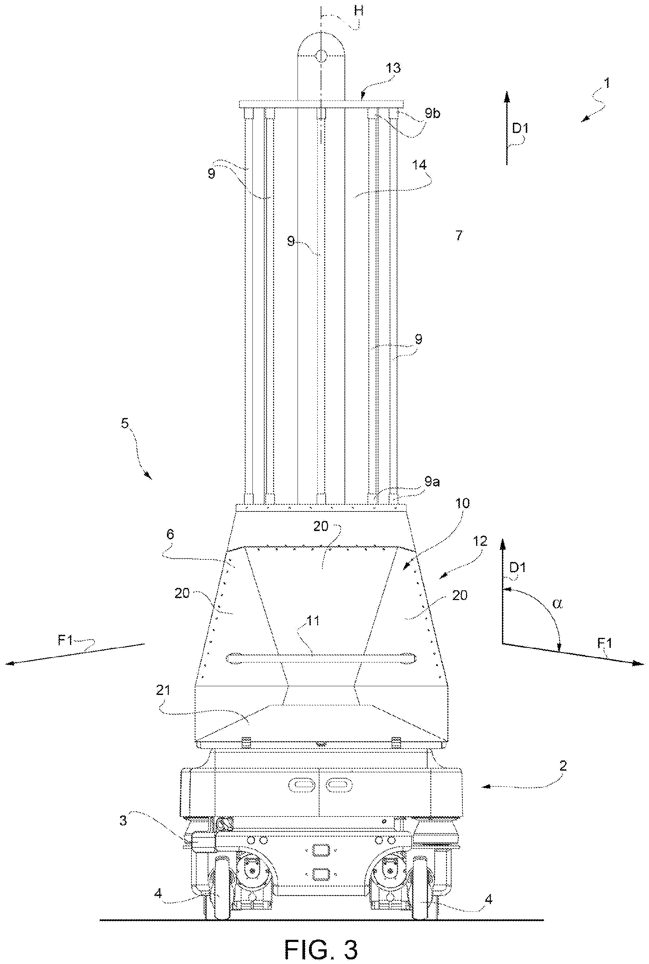

As shown in Figures 3, 4 and 5, each ultraviolet radiation generator **10** is configured to emit an ultraviolet radiation beam **Fl** which propagates along a lateral direction, relatively to the trolley **3,** and which forms with the direction **D1**, on a vertical plane, an angle **α** to radiate ultraviolet radiations to a corresponding portion of the surrounding environment.

As described in more detail below, the direction of the beam **F1** and, hence, the angle **α** is adjustable so that the radiation of the beam **Fl** reaches surfaces of the environment that would not be reached if the direction of the radiation beam **F1** were fixed, like in the state-of-the-art robots. This significantly increases the effectiveness of the robot **1** in eliminating viruses and bacteria.

In particular, in a preferred embodiment, the ultraviolet radiation generator **10** comprises an ultraviolet radiation generator tube **11** extending along a second direction **D2** and a concave reflector **12** associated with the generator tube **11** and at least partially movable so as to allow the radiation direction of the beam **F1** to be varied.

Preferably, like in the example of the shown figures, the direction **D2** along which the generator tube **11** extends is perpendicular to the first direction **D1** and, in particular, is horizontal.

The reflector **12** has a concave structure and is delimited by a plurality of reflecting walls **20,** one or more of which are each fixed relative to the frame **6,** and at least one of which, denoted with the reference numeral **21,** is movable relative to the fixed reflecting walls **20** and the frame **6.**

In the example shown, the reflector **12** has a trapezoidal-shaped cross-section in a plane perpendicular to the direction **D2.**

The movable reflecting wall **21** consists of the lower wall of the reflector **12** and has a generally trapezoidal shape, in which the minor base faces the bottom of the reflector **12** and the major base extends along, or at, an outer peripheral edge of the reflector **12.**

The movable reflecting wall **21** is hinged to the frame **6** at its major base and is associated with a pusher (not shown), which acts on an inner face (not visible in the figures) of the movable reflecting part **21** in order to rotate the movable reflecting wall **21** around the major base so as to vary its inclination. Preferably, the major side of the movable reflecting wall **21** is parallel to the direction **D2,** i.e., to the generator tube **11.**

In different embodiments not shown, the reflector **12** comprises more than one movable reflecting wall **21** so as to allow even greater control of the direction of the radiation beam **F1** radiated by the generator tube **11.**

In a different embodiment not shown, the ultraviolet radiation generator **10** comprises additional generator tubes similar to the generator tube **11,** preferably but not necessarily, parallel to the generator tube **11.**

As shown in the Figures, the ultraviolet radiation device **5** further comprises an additional ultraviolet radiation generator 7, which is mounted on the frame **6** of the ultraviolet radiation generator **10** and extends from an upper wall **8** of the frame **6** along the axis **H** parallel to the direction **D1**.

The ultraviolet radiation generator **7** is configured to radiate an ultraviolet radiation with an intensity that is substantially angularly constant around the axis **H** and in a radiation direction that is mainly radial to the axis **H.**

In the example shown in Figures 1 to 5, the ultraviolet radiation generator **7** comprises a plurality of rectilinear ultraviolet tubes **9** arranged mutually angularly equispaced around the axis **H** so as to form a cage-shaped structure that radiates substantially uniformly in a mainly radial direction all around the axis **H.**

Lower ends **9a** of the ultraviolet tubes **9** are carried by the wall **8,** while upper ends **9b** of the ultraviolet tubes **9** are carried by a flat circular wall **13** perpendicular to the axis **H.** The ultraviolet radiation generator **7** further comprises a reflector **14** arranged in the cage-shaped structure and designed to reflect the ultraviolet radiation radiated by the ultraviolet tubes **9.** The reflector **14** has a polygonal cross-section relative to the axis **H.**

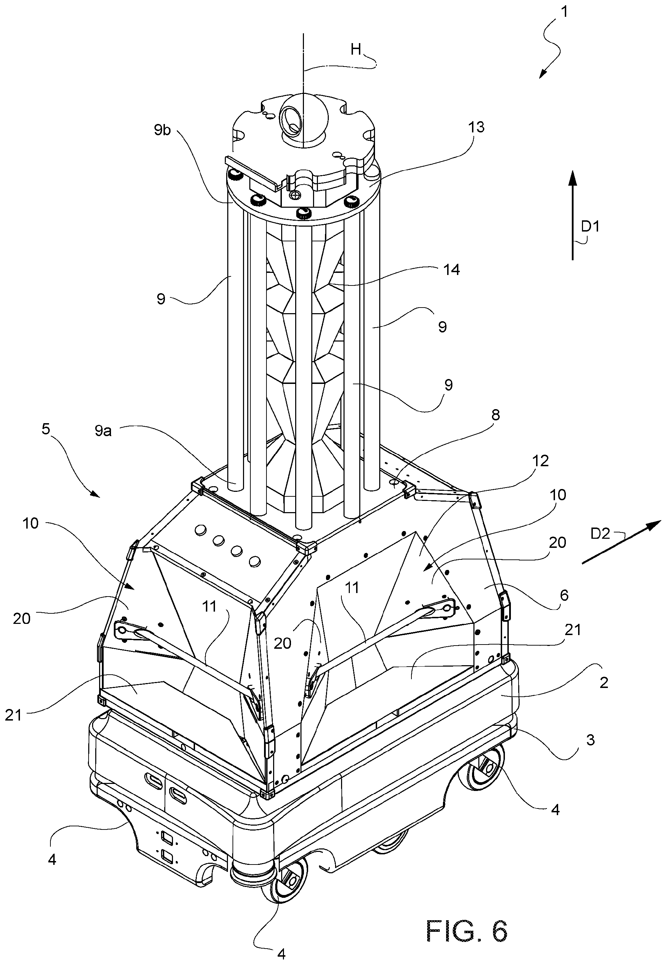

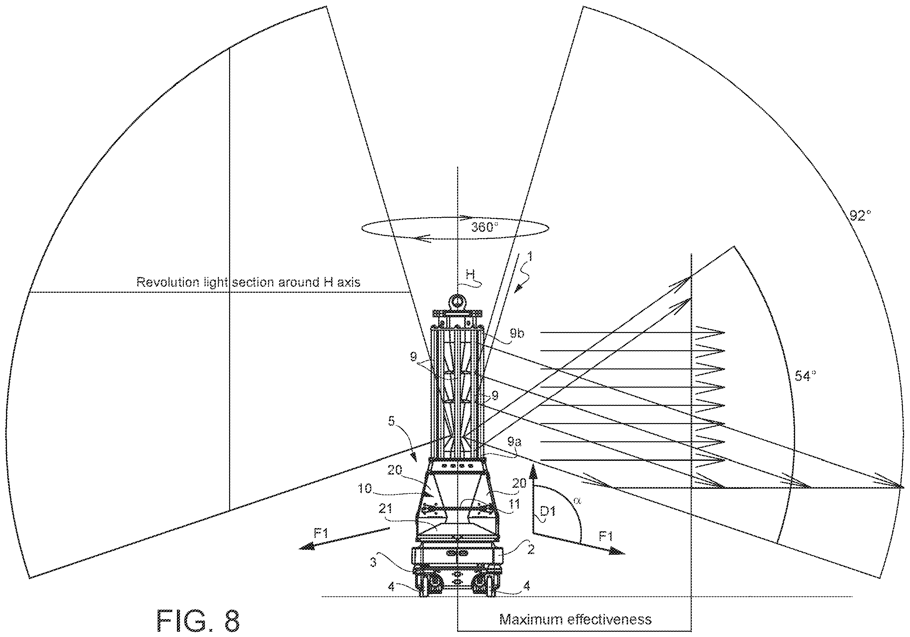

In the alternative embodiment shown in Figures 6 to 8, the reflector **14** comprises a plurality of polyhedral reflectors that are stacked and aligned along the axis **H.** The particular arrangement of the reflectors **14** causes the ultraviolet light radiated by the ultraviolet tubes **9** and reflected by the reflectors **14** to be directed not only in a direction substantially radial to the axis **H,** but to propagate all around the axis **H** also in directions inclined with respect to the axis **H** so as to form, in a vertical plane, an radiation angular sector having a given angular amplitude, which is a function of the inclination of the faces laterally delimiting the reflectors **14.** Preferably, as shown in Figure 8, the angular amplitude of the radiation sector is approximately 90°.

## Claims

1. An automated guided robot **(1)** for environmental sanitation, comprising an automated guided vehicle **(2)** with a motorised trolley **(3)** to move around in an environment to be sanitised; the robot **(1)** further comprises an ultraviolet radiation device **(5)** comprising at least one ultraviolet radiation generator **(10)** mounted on the trolley **(3)** to radiate an ultraviolet radiation beam (**F1**) in an radiation direction mainly directed laterally with respect to the trolley **(3);** the radiation direction of the ultraviolet radiation (**F1**) is adjustable to vary a radiated area of the environment; the ultraviolet radiation generator **(10)** comprises at least one ultraviolet radiation generator tube **(11)** and a reflector **(12)** associated with the ultraviolet radiation generator tube **(11)** and is at least partially movable to vary a radiation direction of the ultraviolet radiation generator tube **(11); characterised in that** the reflector **(12)** is concave-shaped and comprises one or more stationary reflecting walls **(20)** mounted stationary relatively to the ultraviolet radiation generator tube **(11),** and at least one movable reflecting wall **(21)** mounted to move relatively to the stationary reflecting wall(s) **(20).**

2. The robot **(1)** of claim **1,** wherein the radiation direction of the ultraviolet radiation beam **(F1)** forms, with a vertical axis **(H),** in a vertical plane, an angle (***α***) adjustable to vary the radiated area of the environment.

3. The robot (1) of claim **1** or **2,** further comprising an actuator associated to the movable reflecting wall **(21)** and designed to vary an inclination of the movable reflecting wall **(21)** to vary the radiation direction of the ultraviolet radiation beam (**F1**).

4. The robot **(1)** of claim **3,** wherein the movable reflecting wall **(21)** is generally trapezoidal-shaped with a minor base facing a bottom of the concave-shaped reflector **(12)** and a major base arranged along, or at, an outer peripheral edge of the concave-shaped reflector **(12);** the actuator is configured to rotate the movable reflecting wall **(21)** about the major base of the movable reflecting wall **(21)** to adjust the radiation direction of the ultraviolet radiation beam **(F1).**

5. The robot **(1)** of any one of the preceding claims **2** to **4,** wherein the ultraviolet radiation device **(5)** comprises a frame **(6)** carrying the ultraviolet radiation generator **(10);** the frame **(6)** is delimited by a plurality of sides, one or more of which carry respective ultraviolet radiation generators **(10)** to generate ultraviolet radiation beams that propagate from the respective sides.

6. The robot **(1)** of any one of the preceding claims, comprising a further ultraviolet radiation generator **(7),** which is carried by the trolley **(3),** is arranged above the ultraviolet radiation generator **(10),** and extends along the vertical axis **(H)** to radiate an ultraviolet radiation beam all around the vertical axis **(H)** in a stationary direction mainly radial to the vertical axis **(H).**

7. The robot **(1)** of claim **6,** wherein the further ultraviolet radiation generator **(7)** comprises a plurality of rectilinear ultraviolet radiation generator tubes **(9),** which are arranged angularly mutually equispaced around the vertical axis **(H)** to form a cage-shaped structure, and a reflector **(14)** arranged inside the cage-shaped structure and designed to reflect the ultraviolet radiation beam from the ultraviolet radiation generator tubes **(9)** to uniformly radiate the space around the vertical axis **(H).**

8. The robot **(1)** according to claim **7,** wherein the reflector **(14)** comprises a plurality of polyhedral elements stacked along the vertical axis **(H)** so that the further ultraviolet radiation generator **(7)** radiates an ultraviolet radiation beam all around the vertical axis **(H)** both in a direction radial to the vertical axis **(H)** and in a direction inclined relative to the vertical axis **(H).**

## Patentansprüche

1. Automatisch geführter Roboter (1) zur Umweltsanierung, der ein automatisch geführtes Fahrzeug (2) mit einem motorisierten Wagen (3) umfasst, um sich in einer zu sanierenden Umgebung zu bewegen; der Roboter (1) umfasst ferner eine Ultraviolettstrahlungsvorrichtung (5), die mindestens einen Ultraviolettstrahlungsgenerator (10) umfasst, der an dem Wagen (3) angebracht ist, um einen Ultraviolettstrahl (F1) in einer Strahlungsrichtung auszustrahlen, die hauptsächlich seitlich in Bezug auf den Wagen (3) gerichtet ist; die Strahlungsrichtung der Ultraviolettstrahlung (F1) ist einstellbar, um einen bestrahlten Bereich der Umgebung zu variieren; der Ultraviolettstrahlungsgenerator (10) umfasst mindestens eine Ultraviolettstrahlungsgeneratorröhre (11) und einen Reflektor (12), der mit der Ultraviolettstrahlungsgeneratorröhre (11) verbunden ist und mindestens teilweise beweglich ist, um eine Strahlungsrichtung der Ultraviolettstrahlungsgeneratorröhre (11) zu verändern; **dadurch gekennzeichnet, dass** der Reflektor (12) konkav geformt ist und eine oder mehrere stationäre reflektierende Wände (20), die relativ zu der Ultraviolettstrahlungsgeneratorröhre (11) stationär angebracht sind, und mindestens eine bewegliche reflektierende Wand (21) umfasst, die so angebracht ist, dass sie sich relativ zu der/den stationären reflektierenden Wand/Wänden (20) bewegt.

2. Roboter (1) nach Anspruch 1, wobei die Strahlungsrichtung des ultravioletten Strahlenbündels (F1) mit einer vertikalen Achse (H) in einer vertikalen Ebene einen Winkel (α) bildet, der einstellbar ist, um die bestrahlte Fläche der Umgebung zu variieren.

3. Roboter (1) nach einem der Ansprüche 1 oder 2, ferner umfassend einen Aktuator, der mit der beweglichen reflektierenden Wand (21) verbunden ist und dazu dient, eine Neigung der beweglichen reflektierenden Wand (21) zu verändern, um die Strahlungsrichtung des ultravioletten Strahlungsbündels (F1) zu verändern.

4. Roboter (1) nach Anspruch 3, wobei die bewegliche reflektierende Wand (21) im Allgemeinen trapezförmig ist, mit einer kleineren Basis, die einem Boden des konkav geformten Reflektors (12) zugewandt ist, und einer größeren Basis, die entlang oder an einer äußeren Umfangskante des konkav geformten Reflektors (12) angeordnet ist; wobei der Aktuator so konfiguriert ist, dass er die bewegliche reflektierende Wand (21) um die größere Basis der beweglichen reflektierenden Wand (21) dreht, um die Strahlungsrichtung des ultravioletten Strahls (F1) einzustellen.

5. Roboter (1) nach einem der vorangehenden Ansprüche 2 bis 4, wobei die Ultraviolettstrahlungsvorrichtung (5) einen Rahmen (6) umfasst, der den Ultraviolettstrahlungsgenerator (10) trägt; der Rahmen (6) wird durch eine Vielzahl von Seiten begrenzt, von denen eine oder mehrere entsprechende Ultraviolettstrahlungsgeneratoren (10) tragen, um Ultraviolettstrahlungsbündel zu erzeugen, die sich von den entsprechenden Seiten ausbreiten.

6. Roboter (1) nach einem der vorhergehenden Ansprüche, umfassend einen weiteren Ultraviolettstrahlungsgenerator (7), der von dem Wagen (3) getragen wird, oberhalb des Ultraviolettstrahlungsgenerators (10) angeordnet ist und sich entlang der vertikalen Achse (H) erstreckt, um einen Ultraviolettstrahl rund um die vertikale Achse (H) in einer stationären Richtung hauptsächlich radial zur vertikalen Achse (H) auszustrahlen.

7. Roboter (1) nach Anspruch 6, wobei der weitere Ultraviolettstrahlungsgenerator (7) eine Vielzahl von geradlinigen Ultraviolettstrahlungsgeneratorröhren (9) umfasst, die winkelmäßig zueinander gleich beabstandet um die vertikale Achse (H) angeordnet sind, um eine käfigförmige Struktur zu bilden, und einen Reflektor (14), der innerhalb der käfigförmigen Struktur angeordnet ist und so gestaltet ist, dass er den Ultraviolettstrahlungsstrahl von den Ultraviolettstrahlungsgeneratorröhren (9) reflektiert, um den Raum um die vertikale Achse (H) gleichmäßig zu bestrahlen.

8. Roboter (1) nach Anspruch 7, wobei der Reflektor (14) eine Vielzahl von polyedrischen Elementen umfasst, die entlang der vertikalen Achse (H) gestapelt sind, sodass der weitere Ultraviolettstrahlungsgenerator (7) einen Ultraviolettstrahl rund um die vertikale Achse (H) sowohl in einer Richtung radial zur vertikalen Achse (H) als auch in einer Richtung relativ zur vertikalen Achse (H) geneigt abstrahlt.

## Revendications

1. Robot (1) à guidage automatique pour l'assainissement de l'environnement, comprenant un véhicule à guidage automatique (2) avec un chariot motorisé (3) pour se déplacer dans un environnement à assainir ; le robot (1) comprend en outre un dispositif à rayonnement ultraviolet (5) comprenant au moins un générateur de rayonnement ultraviolet (10) monté sur le chariot (3) afin de rayonner un faisceau de rayonnement ultraviolet (F1) dans une direction de rayonnement principalement dirigée latéralement par rapport au chariot (3) ; la direction de rayonnement du rayonnement ultraviolet (F1) est réglable afin de modifier une zone rayonnée de l'environnement ; le générateur de rayonnement ultraviolet (10) comprend au moins un tube de générateur de rayonnement ultraviolet (11) et un réflecteur (12) associé avec le tube de générateur de rayonnement ultraviolet (11) et est au moins partiellement mobile pour modifier une direction de rayonnement du tube de générateur de rayonnement ultraviolet (11) ; **caractérisé en ce que** le réflecteur (12) est de forme concave et comprend une ou plusieurs parois réfléchissantes fixes (20) montées de manière fixe par rapport au tube de générateur de rayonnement ultraviolet (11), et au moins une paroi réfléchissante mobile (21) montée pour se déplacer par rapport à la (aux) paroi(s) réfléchissante(s) fixe(s) (20).

2. Robot (1) selon la revendication 1, dans lequel la direction de rayonnement du faisceau de rayonnement ultraviolet (F1) forme, avec un axe vertical (H), dans un plan vertical, un angle (*α*) réglable afin de modifier la zone rayonnée de l'environnement.

3. Robot (1) selon la revendication 1 ou 2, comprenant en outre un actionneur associé à la paroi réfléchissante mobile (21) et conçu pour modifier une inclinaison de la paroi réfléchissante mobile (21) afin de modifier la direction de rayonnement du faisceau de rayonnement ultraviolet (F1).

4. Robot (1) selon la revendication 3, dans lequel la paroi réfléchissante mobile (21) est de forme généralement trapézoïdale avec une base mineure faisant face à un fond du réflecteur de forme concave (12) et une base majeure agencée le long ou au niveau d'un bord périphérique externe du réflecteur de forme concave (12) ; l'actionneur est configuré pour faire tourner la paroi réfléchissante mobile (21) autour de la base majeure de la paroi réfléchissante mobile (21) afin de régler la direction de rayonnement du faisceau de rayonnement ultraviolet (F1).

5. Robot (1) selon l'une quelconque des revendications 2 à 4, dans lequel le dispositif de rayonnement ultraviolet (5) comprend un bâti (6) portant le générateur de rayonnement ultraviolet (10) ; le bâti (6) est délimité par une pluralité de côtés, dont un ou plusieurs porte(nt) les générateurs de rayonnement ultraviolet (10) afin de générer des faisceaux de rayonnement ultraviolet qui se propagent à partir des côtés respectifs.

6. Robot (1) selon l'une quelconque des revendications précédentes, comprenant un autre générateur de rayonnement ultraviolet (7) qui est porté par le chariot (3), est agencé au-dessus du générateur de rayonnement ultraviolet (10) et s'étend le long de l'axe vertical (H) afin de rayonner un faisceau de rayonnement ultraviolet tout autour de l'axe vertical (H) dans une direction fixe principalement radiale par rapport à l'axe vertical (H).

7. Robot (1) selon la revendication 6, dans lequel le générateur de rayonnement ultraviolet supplémentaire (7) comprend une pluralité de tubes de générateur de rayonnement ultraviolet rectilignes (9) qui sont agencés de manière angulaire mutuellement à équidistance autour de l'axe vertical (H) afin de former une structure en forme de cage, et un réflecteur (14) agencé à l'intérieur de la structure en forme de cage et conçu pour réfléchir le faisceau de rayonnement ultraviolet des tubes de générateur de rayonnement ultraviolet (9) afin de rayonner uniformément l'espace autour de l'axe vertical (H).

8. Robot (1) selon la revendication 7, dans lequel le réflecteur (14) comprend une pluralité d'éléments polyèdres empilés le long de l'axe vertical (H) de sorte que le générateur de rayonnement ultraviolet supplémentaire (7) rayonne un faisceau de rayonnement ultraviolet tout autour de l'axe vertical (H) à la fois dans une direction radiale par rapport à l'axe vertical (H) et dans une direction inclinée par rapport à l'axe vertical (H).
